(19) **Europäisches Patentamt / European Patent Office / Office européen des brevets**

(11) **EP 2 175 907 B1**

(12) **EUROPEAN PATENT SPECIFICATION**

(45) Date of publication and mention
of the grant of the patent:
**20.07.2016 Bulletin 2016/29**

(21) Application number: **08762418.5**

(22) Date of filing: **20.06.2008**

(51) Int Cl.:
***A61M 1/00*** *(2006.01)*

(86) International application number:
**PCT/GB2008/002101**

(87) International publication number:
**WO 2009/019415 (12.02.2009 Gazette 2009/07)**

(54) **DETERMINING FLOW RATE**

FLUSSRATENBESTIMMUNG

DÉTERMINATION D'UN DÉBIT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.08.2007 GB 0715264**

(43) Date of publication of application:
**21.04.2010 Bulletin 2010/16**

(73) Proprietor: **Smith & Nephew PLC**
**London WC2N 6LA (GB)**

(72) Inventors:
• **TURNER, Jake**
**Cambridge CB23 3UG (GB)**

• **GORDON, Benjamin**
**Cambridge CB1 9HL (GB)**

(74) Representative: **Smith & Nephew**
**Group Research Centre**
**York Science Park**
**Heslington**
**York YO10 5DF (GB)**

(56) References cited:
**WO-A-03/030966     US-A- 4 930 997**
**US-A1- 2007 016 152**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to apparatus and a method for the application of topical negative pressure (TNP) therapy to wounds. In particular, but not exclusively, the present invention relates to a method and apparatus for determining a flow rate generated by a pump without use of a specific flow meter.

There is much prior art available relating to the provision of apparatus and methods of use thereof for the application of TNP therapy to wounds together with other therapeutic processes intended to enhance the effects of the TNP therapy. Examples of such prior art include those listed and briefly described below.

TNP therapy assists in the closure and healing of wounds by reducing tissue oedema; encouraging blood flow and granulation of tissue; removing excess exudates and may reduce bacterial load and thus, infection to the wound. Furthermore, TNP therapy permits less outside disturbance of the wound and promotes more rapid healing.

In our co-pending International patent application, WO 2004/037334, apparatus, a wound dressing and a method for aspirating, irrigating and cleansing wounds are described. In very general terms, this invention describes the treatment of a wound by the application of topical negative pressure (TNP) therapy for aspirating the wound together with the further provision of additional fluid for irrigating and/or cleansing the wound, which fluid, comprising both wound exudates and irrigation fluid, is then drawn off by the aspiration means and circulated through means for separating the beneficial materials therein from deleterious materials. The materials which are beneficial to wound healing are recirculated through the wound dressing and those materials deleterious to wound healing are discarded to a waste collection bag or vessel.

In our co-pending International patent application, WO 2005/046760, apparatus, a wound dressing and a method for cleansing a wound using aspiration, irrigation and cleansing wounds are described. Again, in very general terms, the invention described in this document utilises similar apparatus to that in WO 2004/037334 with regard to the aspiration, irrigation and cleansing of the wound, however, it further includes the important additional step of providing heating means to control the temperature of that beneficial material being returned to the wound site/dressing so that it is at an optimum temperature, for example, to have the most efficacious therapeutic effect on the wound.

In our co-pending International patent application, WO 2005/105180, apparatus and a method for the aspiration, irrigation and/or cleansing of wounds are described. Again, in very general terms, this document describes similar apparatus to the two previously mentioned documents hereinabove but with the additional step of providing means for the supply and application of physiologically active agents to the wound site/dressing to promote wound healing.

However, the above apparatus and methods are generally only applicable to a patient when hospitalised as the apparatus is complex, needing people having specialist knowledge in how to operate and maintain the apparatus, and also relatively heavy and bulky, not being adapted for easy mobility outside of a hospital environment by a patient, for example.

Some patients having relatively less severe wounds which do not require continuous hospitalisation, for example, but whom nevertheless would benefit from the prolonged application of TNP therapy, could be treated at home or at work subject to the availability of an easily portable and maintainable TNP therapy apparatus.

GB-A-2 307 180 describes a portable TNP therapy unit which may be carried by a patient clipped to belt or harness. It will be appreciated however that there may be certain inaccuracies associated with the provision of a desired flow rate at a wound site. It is therefore desirable to be able to measure flow rate in a TNP system accurately and promptly.

US 2007/0016152 uses a reference airflow to the suction source to provide a means to monitor a change in operation.

It will be appreciated that with prior known pump units a problem is that the flow rate provided by the pump must fall within predetermined desired threshold values. As the pump wears over time or when certain environmental factors change the flow rate provided by the pump can vary which can cause complication or non ideal environments. Also certain known prior art pump systems are noisy during operation or when pressure control is initiated can cause 'quiet' or 'loud' periods. This can be of concern to a user. It will also be appreciated that a lack of smooth speed responses can reduce operational lifetimes as the pump rarely experiences full duty cycles. Still furthermore fluctuations in flow rate can result in pain to a user.

One way to control and monitor flow rate is to utilise one or more flow meters. However these are costly, are prone to error and can be unresponsive to rapid flow rate changes.

It is an aim of the present invention to at least partly mitigate the above-mentioned problems.

It is an aim of embodiments of the present invention to provide a method and apparatus of determining a flow rate generated by a pump of a topical negative pressure (TNP) system.

It is an aim of embodiments of the present invention to provide control of a suction pump of a topical negative pressure system without requiring a flow meter in the system.

The invention is as claimed in the claims.

The invention is comprised in part of an overall apparatus for the provision of TNP therapy to a patient in almost any environment. The apparatus is lightweight, may be mains or battery powered by a rechargeable battery pack contained within a device (henceforth, the term "device" is used to connote a unit which may contain all of the control, power supply, power supply recharging, electronic indicator means and means for initiating and sustaining aspiration functions to a wound and any further necessary functions of a similar nature). When outside the home, for example, the apparatus may provide for an extended period of operation on battery power and in the home, for example, the device may be connected to the mains by a charger unit whilst still being used and operated by the patient.

The overall apparatus of which the present invention is a part comprises: a dressing covering the wound and sealing at least an open end of an aspiration conduit to a cavity formed over the wound by the dressing; an aspiration tube comprising at least one lumen therethrough leading from the wound dressing to a waste material canister for collecting and holding wound exudates/waste material prior to disposal; and, a power, control and aspiration initiating and sustaining device associated with the waste canister.

The dressing covering the wound may be any type of dressing normally employed with TNP therapy and, in very general terms, may comprise, for example, a semi-permeable, flexible, self-adhesive drape material, as is known in the dressings art, to cover the wound and seal with surrounding sound tissue to create a sealed cavity or void over the wound. There may aptly be a porous barrier and support member in the cavity between the wound bed and the covering material to enable an even vacuum distribution to be achieved over the area of the wound. The porous barrier and support member being, for example, a gauze, a foam an inflatable bag or known wound contact type material resistant to crushing under the levels of vacuum created and which permits transfer of wound exudates across the wound area to the aspiration conduit sealed to the flexible cover drape over the wound.

The aspiration conduit may be a plain flexible tube, for example, having a single lumen therethrough and made from a plastics material compatible with raw tissue, for example. However, the aspiration conduit may have a plurality of lumens therethrough to achieve specific objectives relating to the invention. A portion of the tube sited within the sealed cavity over the wound may have a structure to enable continued aspiration and evacuation of wound exudates without becoming constricted or blocked even at the higher levels of the negative pressure range envisaged.

It is envisaged that the negative pressure range for the apparatus embodying the present invention may be between about -50 mmHg and -200 mmHg (note that these pressures are relative to normal ambient atmospheric pressure thus, -200 mmHg would be about 560 mmHg in practical terms). Aptly, the pressure range may be between about -75 mmHg and -150 mmHg. Alternatively a pressure range of upto -75 mmHg, upto -80 mmHg or over -80 mmHg can be used. Also aptly a pressure range of below - 75 mmHg could be used. Alternatively a pressure range of over -100 mmHg could be used or over -150 mmHg.

The aspiration conduit at its distal end remote from the dressing may be attached to the waste canister at an inlet port or connector. The device containing the means for initiating and sustaining aspiration of the wound/dressing may be situated between the dressing and waste canister, however, in a preferred embodiment of the apparatus embodying the present invention, the device may aspirate the wound/dressing via the canister thus, the waste canister may preferably be sited between the wound/dressing and device.

The aspiration conduit at the waste material canister end may preferably be bonded to the waste canister to prevent inadvertent detachment when being caught on an obstruction, for example.

The canister may be a plastics material moulding or a composite unit comprising a plurality of separate mouldings. The canister may aptly be translucent or transparent in order to visually determine the extent of filling with exudates. However, the canister and device may in some embodiments provide automatic warning of imminent canister full condition and may also provide means for cessation of aspiration when the canister reaches the full condition.

The canister may be provided with filters to prevent the exhaust of liquids and odours therefrom and also to prevent the expulsion of bacteria into the atmosphere. Such filters may comprise a plurality of filters in series. Examples of suitable filters may comprise hydrophobic filters of $0.2\mu$m pore size, for example, in respect of sealing the canister against bacteria expulsion and 1 $\mu$m against liquid expulsion.

Aptly, the filters may be sited at an upper portion of the waste canister in normal use, that is when the apparatus is being used or carried by a patient the filters are in an upper position and separated from the exudate liquid in the waste canister by gravity. Furthermore, such an orientation keeps the waste canister outlet or exhaust exit port remote from the exudate surface.

Aptly the waste canister may be filled with an absorbent gel such as ISOLYSEL (trade mark), for example, as an added safeguard against leakage of the canister when full and being changed and disposed of. Added advantages of a gel matrix within the exudate storing volume of the waste canister are that it prevents excessive movement, such as slopping, of the liquid, minimises bacterial growth and minimises odours.

The waste canister may also be provided with suitable means to prevent leakage thereof both when detached from the device unit and also when the aspiration conduit is detached from the wound site/dressing.

The canister may have suitable means to prevent emptying by a user (without tools or damage to the canister) such that a full or otherwise end-of-life canister may only be disposed of with waste fluid still contained.

The device and waste canister may have mutually complementary means for connecting a device unit to a waste canister whereby the aspiration means in the device unit automatically connects to an evacuation port on the waste canister such that there is a continuous aspiration path from the wound site/dressing to an exhaust port on the device.

Aptly, the exhaust port from the fluid path through the apparatus is provided with filter means to prevent offensive odours from being ejected into the atmosphere.

In general terms the device unit comprises an aspirant pump; means for monitoring pressure applied by the aspirant pump; a control system which controls the aspirant pump in response to signals from sensors such as the pressure monitoring means, for example, and which control system also controls a power management system with regard to an on-board battery pack and the charging thereof and lastly a user interface system whereby various functions of the device such as pressure level set point, for example, may be adjusted (including stopping and starting of the apparatus) by a user. The device unit may contain all of the above features within a single unified casing.

In view of the fact that the device unit contains the majority of the intrinsic equipment cost therein ideally it will also be able to survive impact, tolerate cleaning in order to be reusable by other patients.

In terms of pressure capability the aspiration means may be able to apply a maximum pressure drop of at least -200 mmHg to a wound site/dressing. The apparatus is capable of maintaining a predetermined negative pressure even under conditions where there is a small leak of air into the system and a high exudate flow.

The pressure control system may prevent the minimum pressure achieved from exceeding for example -200 mmHg so as not to cause undue patient discomfort. The pressure required may be set by the user at a number of discreet levels such as -50, -75, -100, -125, -150, -175 mmHg, for example, depending upon the needs of the wound in question and the advice of a clinician. Thus suitable pressure ranges in use may be from -25 to -80 mmHg, or -50 to -76 mmHg, or -50 to -75 mmHg as examples. The control system may also advantageously be able to maintain the set pressure within a tolerance band of +/- 10 mmHg of the set point for 95% of the time the apparatus is operating given that leakage and exudation rates are within expected or normal levels.

Aptly, the control system may trigger alarm means such as a flashing light, buzzer or any other suitable means when various abnormal conditions apply such as, for example: pressure outside set value by a large amount due to a gross leak of air into system; duty on the aspiration pump too high due to a relatively smaller leakage of air into the system; pressure differential between wound site and pump is too high due, for example, to a blockage or waste canister full.

The apparatus of the present invention may be provided with a carry case and suitable support means such as a shoulder strap or harness, for example. The carry case may be adapted to conform to the shape of the apparatus comprised in the joined together device and waste canister. In particular, the carry case may be provided with a bottom opening flap to permit the waste canister to be changed without complete removal of the apparatus form the carry case.

The carry case may be provided with an aperture covered by a displaceable flap to enable user access to a keypad for varying the therapy applied by the apparatus.

According to a second aspect of the present invention, there is provided apparatus for determining flow rate in a topical negative pressure (TNP) system, comprising:

a pump element, comprising a rotor element that provides negative pressure in a flow path;
a pressure sensor arranged to determine a pressure in the flow path; and a processing unit that determines a pumping speed associated with the pump element and determines flow rate in the flow path using a relationship between the pumping speed and the pressure.

Embodiments of the present invention provide a method and apparatus in which the flow rate in a TNP system may be determined without the need for a flow meter. Flow rate can be calculated using only a pressure sensor which is placed in any one of a number of optional locations in a flow path. This results in a very versatile system. A flow meter could be used to achieve a similar goal but is far more costly than utilisation of a pressure sensor and a mechanism for determining pump speed. The flow rate calculated according to embodiments of the present invention is also highly accurate.

Embodiments of the present invention can provide an alternative flow measurement mechanism in addition to a flow meter in a TNP system. This can be utilised as a safety back up by comparing results and determining that an error has occurred if the results do not match.

Embodiments of the present invention provide increased user confidence and early detection against leaking dressings which can lead to lower power usage.

In order that the present invention may be more fully understood, examples will now be described by way of illustration only with reference to the accompanying drawings, of which:

Figure 1 shows a generalised schematic block diagram showing a general view of an apparatus and the constituent apparatus features thereof;

Figure 2 shows a similar generalised schematic block diagram to Figure 1 and showing fluid paths therein;

Figure 3 shows a generalised schematic block diagram similar to Figure 1 but of a device unit only and showing power paths for the various power consuming/producing features of the apparatus;

Figure 4 shows a similar generalised schematic block diagram to Figure 3 of the device unit and showing control system data paths for controlling the various functions and components of the apparatus;

Figure 5 shows a perspective view of an apparatus;

Figure 6 shows a perspective view of an assembled device unit of the apparatus of Figure 5;

Figure 7 shows an exploded view of the device unit of Figure 6;

Figure 8 shows a partially sectioned side elevation view through the interface between a waste canister and device unit of the apparatus;

Figure 9 shows a cross section through a waste canister of the apparatus of Figures 5 to 8;

Figure 10 illustrates how a pump speed can be measured and selected;

Figure 11 illustrates how pressure generated by a pump can be controlled;

Figure 12 illustrates how flow rate may be calculated without a flow meter; and

Figure 13 illustrates a relationship between flow rate, pressure and pump speed.

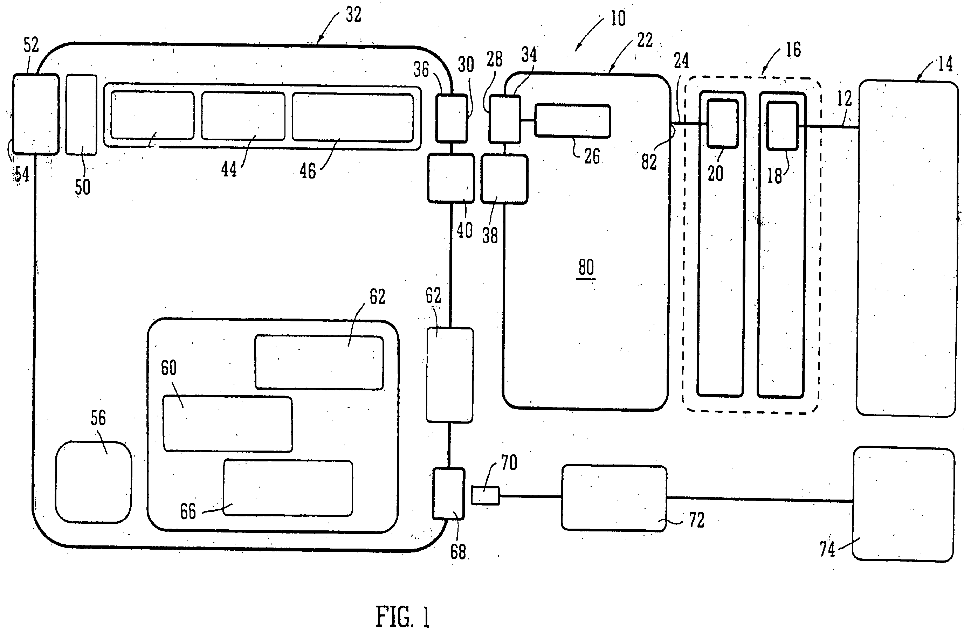

Referring now to Figures 1 to 4 of the drawings and where the same or similar features are denoted by common reference numerals.

Figure 1 shows a generalised schematic view of an apparatus 10 of a portable topical negative pressure (TNP) system. It will be understood that embodiments of the present invention are generally applicable to use in such a TNP system. Briefly, negative pressure wound therapy assists in the closure and healing of many forms of "hard to heal" wounds by reducing tissue oedema; encouraging blood flow and granular tissue formation; removing excess exudate and may reduce bacterial load (and, therefore, infection). In addition the therapy allows for less disturbance of a wound leading to more rapid healing. The TNP system is detailed further hereinafter but in summary includes a portable body including a canister and a device with the device capable of providing an extended period of continuous therapy within at least a one year life span. The system is connected to a patient via a length of tubing with an end of the tubing operably secured to a wound dressing on the patient.

More particularly, as shown in Figure 1, the apparatus comprises an aspiration conduit 12 operably and an outer surface thereof at one end sealingly attached to a dressing 14. The dressing 14 will not be further described here other than to say that it is formed in a known manner from well know materials to those skilled in the dressings art to create a sealed cavity over and around a wound to be treated by TNP therapy with the apparatus of the present invention. The aspiration conduit has an in-line connector 16 comprising connector portions 18, 20 intermediate its length between the dressing 14 and a waste canister 22. The aspiration conduit between the connector portion 20 and the canister 22 is denoted by a different reference numeral 24 although the fluid path through conduit portions 12 and 24 to the waste canister is continuous. The connector portions 18, 20 join conduit portions 12, 24 in a leak-free but disconnectable manner. The waste canister 22 is provided with filters 26 which prevent the escape via an exit port 28 of liquid and bacteria from the waste canister. The filters may comprise a $1\mu m$ hydrophobic liquid filter and a $0.2\mu m$ bacteria filter such that all liquid and bacteria is confined to an interior waste collecting volume of the waste canister 22. The exit port 28 of the waste canister 22 mates with an entry/suction port 30 of a device unit 32 by means of mutually sealing connector portions 34, 36 which engage and seal together automatically when the waste canister 22 is attached to the device unit 32, the waste canister 22 and device unit 32 being held together by catch assemblies 38, 40. The device unit 32 comprises an aspirant pump 44 and an aspirant pressure monitor 46 operably connected together. The aspiration path takes the aspirated fluid which in the case of fluid on the exit side of exit port 28 is gaseous through a silencer system 50 and a final filter 52 having an activated charcoal matrix which ensures that no odours escape with the gas exhausted from the device 32 via an exhaust port 54. The filter 52 material also serves as noise reducing material to enhance the effect of the silencer system 50. The device 32 also contains a battery pack 56 to power the apparatus which battery pack also powers the control system 60 which controls a user interface system 62 controlled via a keypad (not shown) and the aspiration pump 44 via signals from sensors. A power management system 66 is also provided which controls power from the battery pack 56, the recharging thereof and the power requirements of the aspirant pump 44 and other electrically operated components. An electrical connector 68 is provided to receive a power input jack 70 from a SELV power supply 72 connected to a mains supply 74 when the user of the apparatus or the apparatus itself is adjacent a convenient mains power socket.

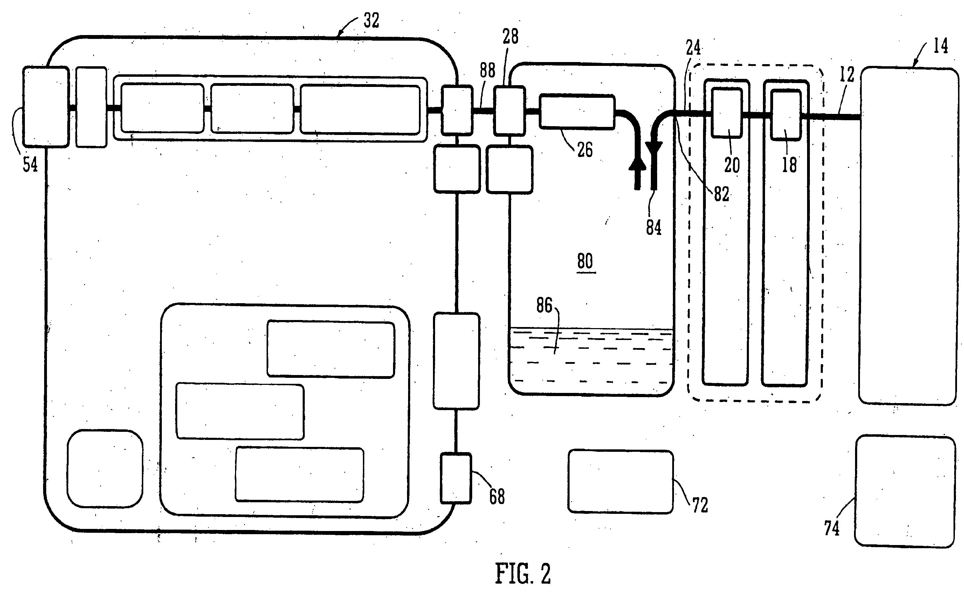

Figure 2 shows a similar schematic representation to Figure 1 but shows the fluid paths in more detail. The wound exudate is aspirated from the wound site/dressing 14 via the conduit 12, the two connector portions 18, 20 and the conduit 24 into the waste canister 22. The waste canister 22 comprises a relatively large volume 80 in the region of 500ml into which exudate from the wound is drawn by the aspiration system at an entry port 82. The fluid 84 drawn into the canister volume 80 is a mixture of both air drawn into the dressing 14 via the semi-permeable adhesive sealing drape (not shown) and liquid 86 in the form of wound exudates. The volume 80 within the canister is also at a lowered pressure and the gaseous element 88 of the aspirated fluids is exhausted from the canister volume 80 via the filters 26 and the waste canister exhaust exit port 28 as bacteria-free gas. From the exit port 28 of the waste canister to the final exhaust port 54 the fluid is gaseous only.

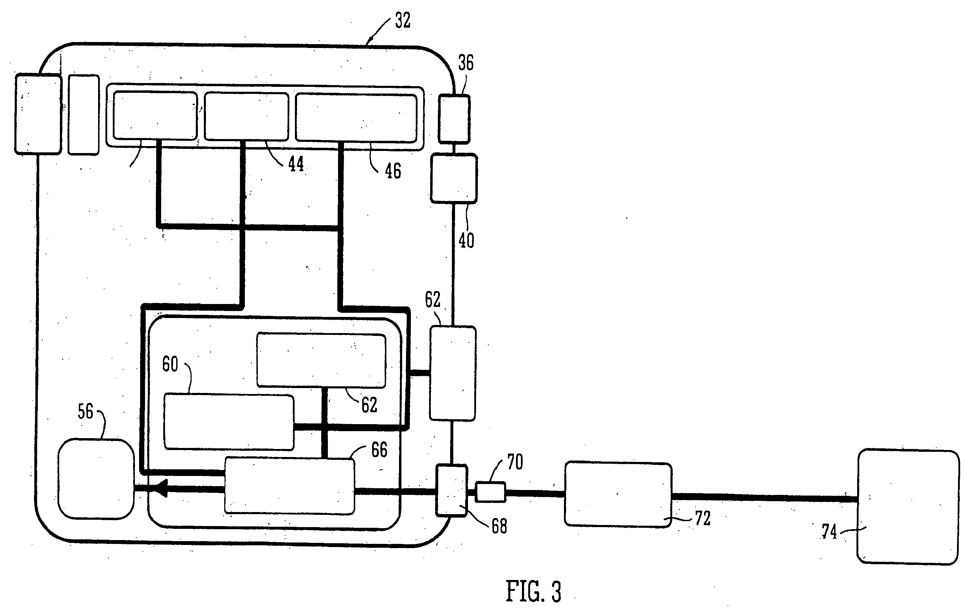

Figure 3 shows a schematic diagram showing only the device portion of the apparatus and the power paths in the device of the apparatus embodying the present invention. Power is provided mainly by the battery pack 56 when the user is outside their home or workplace, for example, however, power may also be provided by an external mains 74 supplied charging unit 72 which when connected to the device 32 by the socket 68 is capable of both operating the device and recharging the battery pack 56 simultaneously. The power management system 66 is included so as to be able to control power of the TNP system. The TNP system is a rechargeable, battery powered system but is capable of being run directly from mains electricity as will be described hereinafter more fully with respect to the further figures. If disconnected from the mains the battery has enough stored charge for approximately 8 hours of use in normal conditions. It will be appreciated that batteries having other associated life times between recharge can be utilised. For example batteries providing less than 8 hours or greater than 8 hours can be used. When connected to the mains the device will run off the mains power and will simultaneously recharge the battery if depleted from portable use. The exact rate of battery recharge will depend on the load on the TNP system. For example, if the wound is very large or there is a significant leak, battery recharge will take longer than if the wound is small and well sealed.

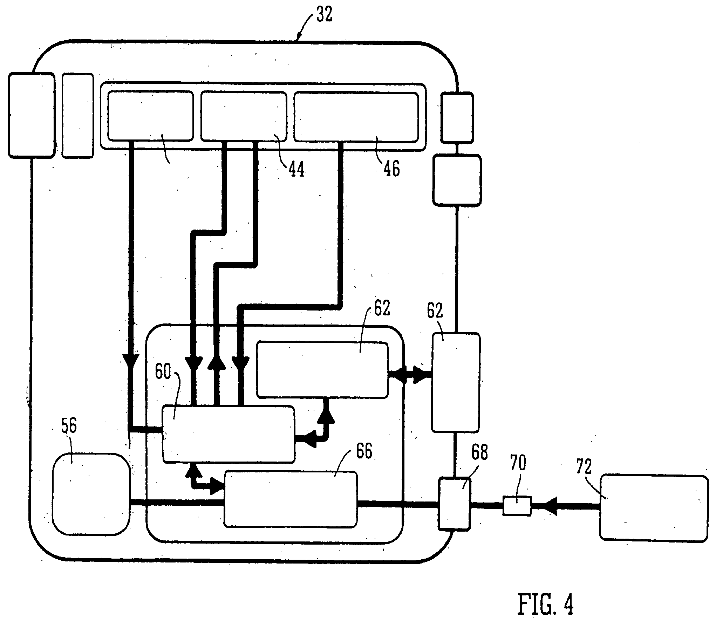

Figure 4 shows the device 32 part of the apparatus embodying the present invention and the data paths employed in the control system for control of the aspirant pump and other features of the apparatus. A key purpose of the TNP system is to apply negative pressure wound therapy. This is accomplished via the pressure control system which includes the pump and a pump control system. The pump applies negative pressure; the pressure control system gives feedback on the pressure at the pump head to the control system; the pump control varies the pump speed based on the difference between the target pressure and the actual pressure at the pump head. In order to improve accuracy of pump speed and hence provide smoother and more accurate application of the negative pressure at a wound site, the pump is controlled by an auxiliary control system. The pump is from time to time allowed to "free-wheel" during its duty cycle by turning off the voltage applied to it. The spinning motor causes a "back electro-motive force" or BEMF to be generated. This BEMF can be monitored and can be used to provide an accurate measure of pump speed.

The speed can thus be adjusted more accurately than can prior art pump systems.

According to embodiments of the present invention, actual pressure at a wound site is not measured but the difference between a measured pressure (at the pump) and the wound pressure is minimised by the use of large filters and large bore tubes wherever practical. If the pressure control measures that the pressure at the pump head is greater than a target pressure (closer to atmospheric pressure) for a period of time, the device sends an alarm and displays a message alerting the user to a potential problem such as a leak.

In addition to pressure control a separate flow control system can be provided. Flow rate can be determined and is used to detect when a canister is full or the tube has become blocked. If the flow falls below a certain threshold, the device sounds an alarm and displays a message alerting a user to the potential blockage or full canister.

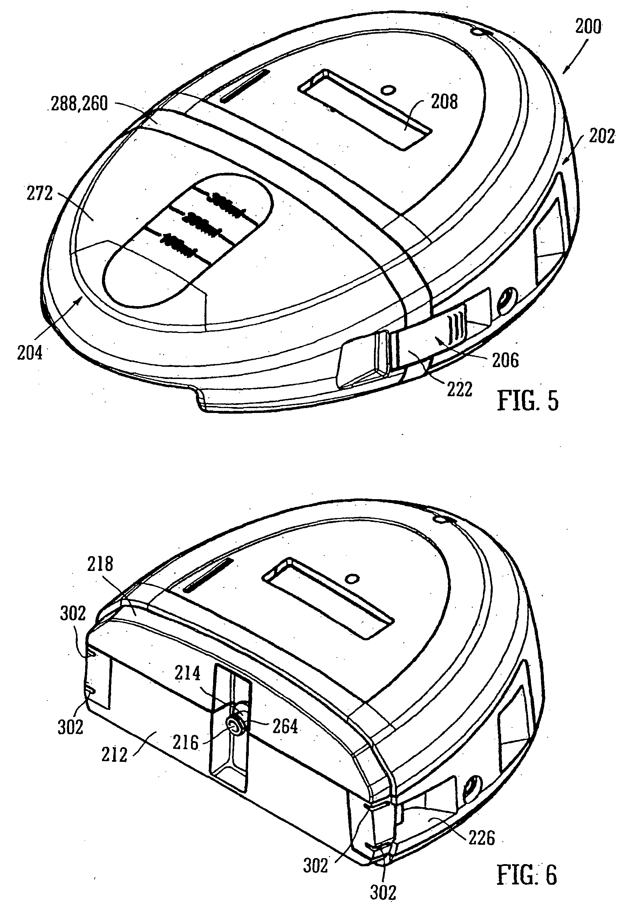

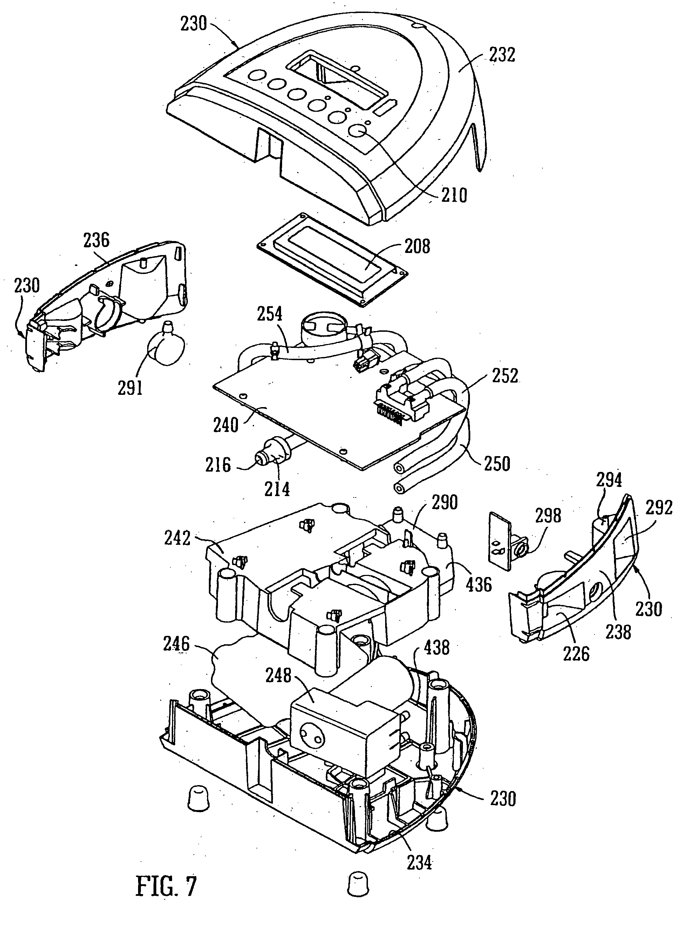

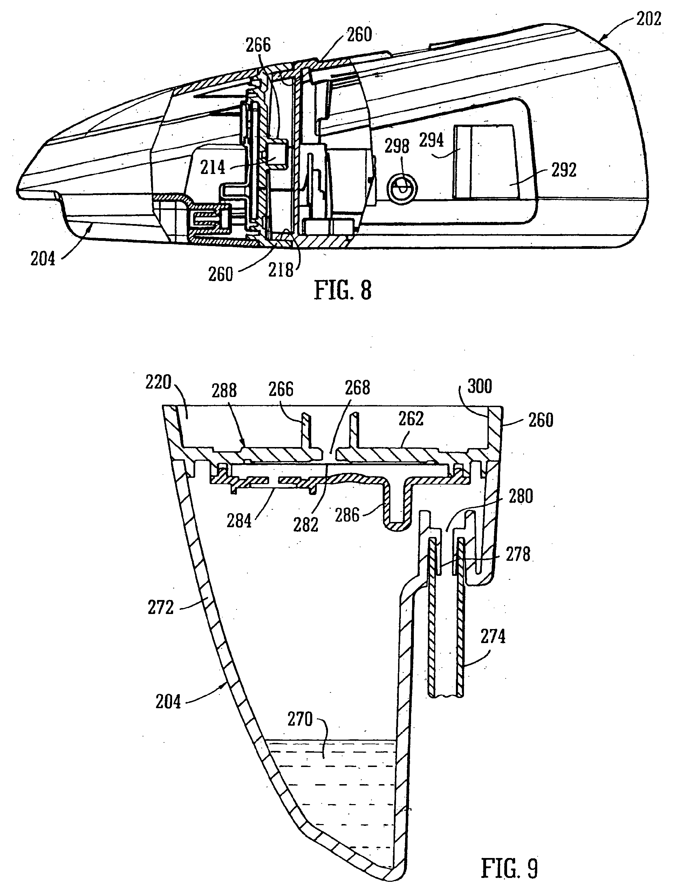

Referring now to Figures 5 to 9 which show various views and cross sections of a preferred embodiment of apparatus 200 embodying the present invention. The preferred embodiment is of generally oval shape in plan and comprises a device unit 202 and a waste canister 204 connected together by catch arrangements 206. The device unit 202 has a liquid crystal display (LCD) 208, which gives text based feedback on the wound therapy being applied, and a membrane keypad 210, the LCD being visible through the membrane of the keypad to enable a user to adjust or set the therapy to be applied to the wound (not shown). The device has a lower, generally transverse face 212 in the centre of which is a spigot 214 which forms the suction/entry port 216 to which the aspiration means (to be described below) are connected within the device unit. The lower edge of the device unit is provided with a rebated peripheral male mating face 218 which engages with a co-operating peripheral female formation 220 on an upper edge of the waste canister 204 (see Figures 8 and 9). On each side of the device 202, clips 222 hinged to the canister 204 have an engaging finger (not shown) which co-operates with formations in recesses 226 in the body of the device unit. From Figure 7 it may be seen that the casing 230 of the device unit is of largely "clamshell" construction comprising front and back mouldings 232, 234, respectively and left-hand and right-hand side inserts 236, 238. Inside the casing 230 is a central chassis 240 which is fastened to an internal moulded structural member 242 and which chassis acts as a mounting for the electrical circuitry and components and also retains the battery pack 246 and aspiration pump unit 248. Various tubing items 250, 252, 254 connect the pump unit 248 and suction/entry port 216 to a final gaseous exhaust via a filter 290. Figure 8 shows a partially sectioned side elevation of the apparatus 200, the partial section being around the junction between the device unit 202 and the waste canister 204, a cross section of which is shown at Figure 9. Theses views show the rebated edge 218 of the male formation on the device unit co-operating with the female portion 220 defined by an upstanding flange 260 around the top face 262 of the waste canister 204. When the waste canister is joined to the device unit, the spigot 214 which has an "O" ring seal 264 therearound sealingly engages with a cylindrical tube portion 266 formed around an exhaust/exit port 268 in the waste canister. The spigot 214 of the device is not rigidly fixed to the device casing but is allowed to "float" or move in its location features in the casing to permit the spigot 214 and seal 264 to move to form the best seal with the bore of the cylindrical tube portion 266 on connection of the waste canister to the device unit. The waste canister 204 in Figure 9 is shown in an upright orientation much as it would be when worn by a user. Thus, any exudate 270 would be in the bottom of the internal volume of waste receptacle portion 272. An aspiration conduit 274 is permanently affixed to an entry port spigot 278 defining an entry port 280 to receive fluid aspirated from a wound (not shown) via the conduit 274. Filter members 282 comprising a $0.2\mu m$ filter and 284 comprising a $1\mu m$ filter are located by a filter retainer moulding 286 adjacent a top closure member or bulkhead 288 the filter members preventing any liquid or bacteria from being drawn out of the exhaust exit port 268 into the pump and aspiration path through to an exhaust and filter unit 290 which is connected to a casing outlet moulding at 291 via an exhaust tube (not shown) in casing side piece 236. The side pieces 236, 238 are provided with recesses 292 having support pins 294 therein to locate a carrying strap (not shown) for use by the patient. The side pieces 230 and canister 204 are also provided with features which prevent the canister and device from exhibiting a mutual "wobble" when connected together. Ribs (not shown) extending between the canister top closure member 288 and the inner face 300 of the upstanding flange 260 locate in grooves 302 in the device sidewalls when canister and device are connected. The casing 230 also houses all of the electrical equipment and control and power management features, the functioning of which was described briefly with respect to Figures 3 and 4 hereinabove. The side piece 238 is provided with a socket member 298 to receive a charging jack from an external mains powered battery charger (both not shown).

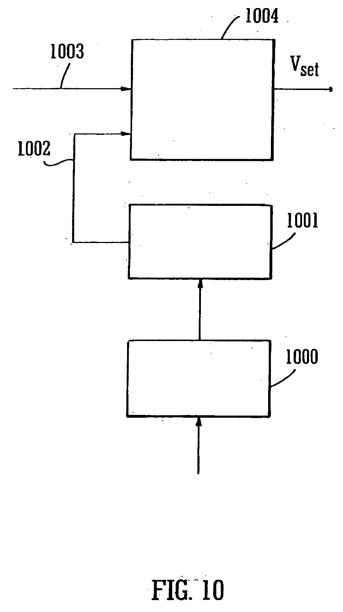

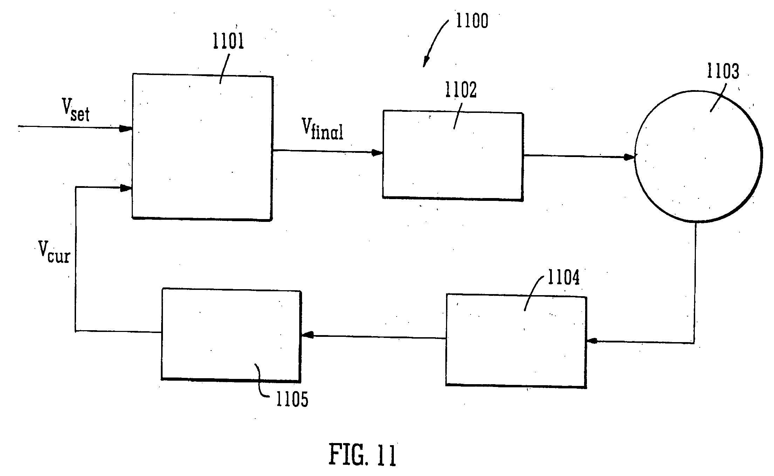

Figures 10 and 11 illustrate how the flow rate provided by a pump of the TNP system can be set, monitored and controlled according to an embodiment of the present invention. As illustrated in Figure 10 a pressure sensor such as a pressure transducer is utilised to measure actual pressure at a pump inlet, which during use will be located at or close to a wound site. It will be appreciated that according to embodiments of the present invention the pressure sensor may be located at some predetermined location along the tube connecting the device unit to the wound site.

Pumping pressure is controlled by an initial pump speed setting system which measures pressure and sets a desired pump speed responsive to the measured pressure and a predetermined pressure set by a user, and a further control loop system in which actual pump speed is monitored and compared with the determined pump speed. Pumping is actually controlled responsive to the comparison.

As illustrated in Figure 10 the pressure determined by the sensor is converted into a digital value in an analogue digital converter 1000 and the value scaled to thereby filter pressure reading before being fed into the control loop to thereby minimise the effect of noise in the reading thereby reducing jitter. This also helps minimise false alarms due to over or under pressure situations.

Pump speed control is achieved by implementing a control loop in hardware or software. The measured scaled pressure provides an input 1002 indicative of the measured pressure into the pressure controller whilst a further input 1003 is provided by a user entering a desired pressure set point via a user interface. The pressure controller 1004 takes the pressure set point and the actual measure of pressure as inputs to deliver a new desired pump speed as its output Vset. The measured pressure values from the pressure transducer are averaged over a certain number of previous readings before feeding a value to the control loop. This minimises jitter and noise and serves as a first dampener of pump response.

The control sequence used for controlling pump response is given below:

> Defines >>> Constants for control loop: kp, ki, t
> Bounds for output: Vmax, Vmin
> Inputs >>> Current pressure value: pv,
> Set point value: sp

Calculate difference: e = sp - pv

$$P = kp * e$$

$$I = I + ki * e * t$$

Verify I is between the Vmin and V max bounds

$$V = P + I$$

Verify V is between the Vmin and V max bounds

Thus the difference between the measured pressure and a desired pressure is calculated and then scaled using experimentally predetermined constants to yield the output value of pump speed Vset. The constants are optimised for best pump response and to minimise pressure overshoot or undershoot. The scaling further dampens the effect of the current pressure difference by taking into account a certain number of previous pressure differences. The control loop is provided to allow only a certain maximum step change in pressure at a time by bounding the output pump speed value within predetermined sensible limits. Thus a sudden change in measured pressure (due to any reason for example the user changing position) or a change in the pressure set point is fed back to the pump drive circuitry incrementally in small steps rather than as a dramatic change.

This mechanism of pump speed control thus results in a better reaction to rapid changes in pressure as the pump does not instantly 'overreact'. Since the pump does not have a drastic reaction to pressure changes the overall 'perceived' noise levels are lower. Gradual adjustment of pump speed also results in lower pump wear and tear which enhances device performance and longevity. Furthermore averaging the pressure transducer readings before feeding them to the control loop reduces the likelihood of false alarms with respect to over or under pressure situations.

Figure 11 illustrates how accurate speed control of a suction pump on the TNP device allows fine control of a negative pressure applied at a wound site and which thus helps reduce noise during device operation and minimises discomfort to the user. The system provides a control loop that periodically turns off power to a pump motor and records an electromotive force (EMF) generated by a freewheeling element such as a rotor of the pump. The measured EMF is used to calculate the actual pump speed and drive signals supplied to the pump can thus be modified to accurately achieve a desired speed.

As illustrated in Figure 11 a control loop 1100 uses the desired and actual pump speeds at a given instant to accurately determine the drive voltage that needs to be applied to the pump in order to accurately achieve final desired speed and thus pressure. The control loop operates by calculating the difference between the desired speed Vset and the current speed Vcur. The pump controller 1101 scales the difference and optionally accumulates the scaled differences from a certain number of previous iterations. The control loop 1101 outputs a value Vfinal for the pump drive voltage that leads to the pump achieving its final desired speed. The scaling constants for the control are determined experimentally prior to operation to ensure acceptable performance of the device (ie. ability to maintain set pressure at specified wound leak rates). The scaling constants can be calculated in various ways however aptly on a startup conditions to provide a predetermined pressure can be applied. A measured actual pressure will indicate operational parameters indicative of pressure change, leaks, wound size and volumes in the waste canister. Scaling constants are then set responsive to these.

The pump control system is responsible for maintaining the pump speed which in turn drives the pressure generated at the wound. The motor speed is controlled by varying a pulse width modulation (PWM) input. The duty cycle of the PWM generator 1102 is controlled responsive to the drive voltage signal Vfinal and the output of the PWM generator is utilised to drive the pump 1103.

The actual speed of the pump is obtained by measuring the terminal voltage across the pump with the current at zero. This is achieved by intermittently turning the pump power off by controlling the PWM generator output. Subsequent to turn off a short period is allowed to wait for the EMF of the freewheeling pump to settle during a certain predetermined time period and thereafter the steady value of the EMF is sampled. This EMF is a direct measure of pump shaft speed. The EMF generated is converted into a digital signal via an analogue digital converter 1104 and then scaled with a scaling unit 1105. The EMF sampling rate is varied according to pump speed to counter the aliasing and to minimise the effect on pump speed. The EMF sampling rate may be reduced at higher pump speed since the inertia of the pump maintains a more constant motion at high pump speeds.

Operation of the control utilised can be summarised by the following control sequence:

```
Pump_Speed_Controller
      Turn pump_enable and PWM off
   Turn pump_enable on after current drops
      Allow EMF to settle
      Sample EMF and estimate current
speed (Vcur)
         new_PWM = PI (Vset, Vcur)
         Enable pump PWM
         New pump duty cycle = new_PWM
   End Motor_Controller
   PI (Vset, Vcur)
         Defines >> constants kp, ki, t
      Inputs >> current motor speed Vcur
               Desired motor speed Vset
      Calculate difference: e = Vset - Vcur
         P = kp * e
         I = I + ki * e * t
   End PI
```

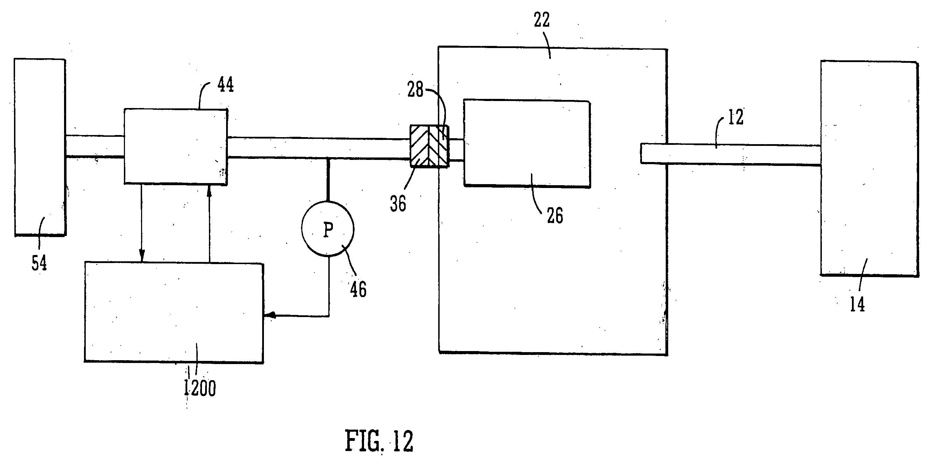

Figure 12 illustrates how embodiments of the present invention can determine a flow rate in a flow path without the need to include a flow meter in a TNP system according to embodiments of the present invention. It will be appreciated that a pressure sensor 46 and pump 44 will often be included in a TNP system and therefore a mechanism for determining flow rate without need for an expensive flow meter utilising units already included in a TNP design produce a favourable system. The system measures the pressure at a pump inlet or some other such desired location as well as determining the speed of the pump via measurement of a back EMF. A known relationship between pump speed, pressure and flow rate can then be used to calculate the flow rate at a location where the pressure sensor is located. Particularly high or low flow rates can be utilised to trigger an alarm condition.

As illustrated in Figure 12 the TNP wound care system includes a pump 44 required to provide the negative pressure. As noted previously the back EMF of the pump can be determined during a free-wheeling mode of operation and the voltage generated is known to be directly proportional to the operating speed of the pump. The operating speed of the pump can thus be measured during device operation.

Again, as noted above, the pump is kept at a proper operating speed using a feedback mechanism including a feedback system 1200 which utilises the measured back EMF as an indication of current pump speed. If a problem occurs with the system, for example a leak occurs, the feedback mechanism detects this as a decrease in pressure at a pressure sensor which, as illustrated in Figure 12, may be located at a pump inlet. The feedback mechanism increases the voltage to the pump to compensate. As the voltage increases, the back EMF will increase in time. The back EMF indicates pumping frequency. This pump frequency, along with the inlet pressure, can be used as a direct indication of system leaks (flow rate).

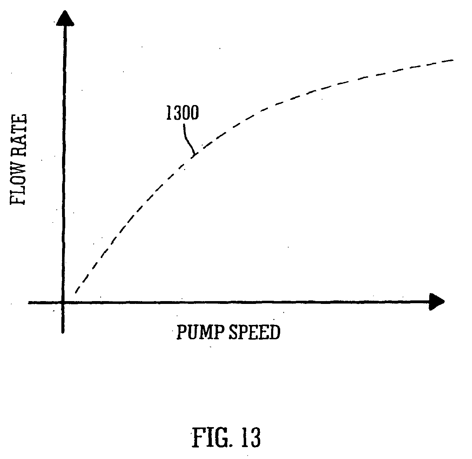

Figure 13 illustrates a known relationship between pump speed, flow rate and pressure difference. It will be appreciated that the particular relationship will be dependent upon the characteristics of the pump system and TNP system generally. To calculate a flow rate from an available pressure and pump speed embodiments of the present invention utilise a "look up table". This table is predetermined experimentally during set up or product development. The look up tables are stored as data in a data store or are set out in software utilised by the TNP system.

As illustrated in Figure 13, for any TNP system a range of values indicating a predetermined relationship 1300 for flow rate can be determined as pump speed varies. This is for a single preset fixed pressure. Similar relationship curves 1300 indicating how flow rate varies with any particular pump speed are determined for many possible fixed pressure values or ranges during TNP system design.

As a result the pressure determined during use by a pressure sensor can be used to select from a look up table of possible pressure values a flow rate and pump speed relationship 1300 for that pressure or pressure range. A pump speed can then be compared to the range and a flow rate read off. It will be appreciated by those skilled in the art that rather than having a flow rate versus pump speed relationship stored in the look up table for a multitude of pressure ranges, a flow rate versus pressure relationship could alternatively be determined for fixed pump speeds. A pump speed would thus be utilised to select a specific look up table storing a relationship between flow rate and pressure.

As indicated in Figure 13, as a rough approximation the relationship between flow rate and pump speed for a fixed pressure difference is linear at low pump speeds and then tends to flatten as the pump has a maximum flow rate it can maintain regardless of speed.

It will be appreciated that the pressure sensor may be placed in alternative locations, such as in front of the canister or in front of the wound, to achieve flow rate calculations.

It will also be appreciated that in accordance with embodiments of the present invention a flow meter may also additionally be utilised in the flow path of the TNP system. Embodiments of the present invention could thus be utilised in conjunction with an alternative flow measurement provided by such a flow meter as a safety back up. As a result embodiments of the present invention provide increased user confidence and may be utilised to detect early leakages of dressings since flow rate can be determined at a further location from where a flow meter is located. This can lead to reduced power usage which causes less drain on internal batteries and lowers cost.

Accurate pump control results in overall lower noise levels during device operation. Specifically abrupt changes in noise are avoided because the pump speed is adjusted frequently and in small steps. Maintaining accurate control of pump speeds can extend pump and battery life. Moreover a steady pump delivers a steady negative pressure thereby minimising patient discomfort.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

**Claims**

1. A method of determining flow rate in a topical negative pressure (TNP) system, comprising the steps of:

    determining a pumping speed associated with a pump element (44) of a TNP system:

    determining a pressure associated with a flow path associated with the pump element; and **characterised by**
    determining flow rate in the flow path by utilising a look up table using a relationship between flow rate, pumping speed and pressure.

2. The method as claimed in claim 1, further comprising the steps of:

    determining the pressure by measuring pressure via a pressure sensor.

**3.** The method as claimed in claim 1 or claim 2 wherein the flow rate comprises a load on the pump element (44).

**4.** The method as claimed in any one of claims 1 to 3 wherein the step of determining the pumping speed comprises the steps of:

> determining an electromotive force (EMF) generated by a free-wheeling element of the pump element (44); and
> calculating the pumping speed responsive to the generated EMF.

**5.** The method as claimed in any preceding claim, further comprising the steps of:

> determining if the determined flow rate is within a predetermined operating range; and
> generating an audible and/or visible alarm cue if the determined flow rate exceeds an upper range limit or falls below a lower range limit.

**6.** The method as claimed in any one of claims 1 to 5, further comprising the steps of:

> from time to time, varying pump speed responsive to the determined flow rate;
> re-determining the pressure subsequent to each step of varying pump speed; and
> re-determining flow rate in the flow path.

**7.** The method as claimed in any preceding claim wherein the step of determining flow rate comprises the steps of:

> determining flow rate at an inlet location of the pump element (44).

**8.** The method as claimed in any preceding claim, further comprising determining flow rate in the flow path by the steps of:

> determining a pump speed or pressure;
> indexing the look-up table responsive to the determined pump speed or pressure; and
> determining a flow rate by indexing a flow rate value associated with a value of a remainder of the pump speed or pressure in the indexed look-up table.

**9.** Apparatus for determining flow rate in a topical negative pressure (TNP) system, comprising:

> a pump element (44), comprising a rotor element that provides negative pressure in a flow path;
> a pressure sensor arranged to determine a pressure in the flow path; and **characterised in that** it further comprises
a processing unit that determines a pumping speed associated with the pump element (44)_and determines flow rate in the flow path utilising a look up table_ using a relationship between flowrate, pumping speed and pressure.

**10.** The apparatus as claimed in claim 9 wherein the flow path comprises:

> a connection tube connecting the pump element (44) to a canister (22) of the TNP system; and
> an aspirant tube connected to the canister (22) and locatable at a wound site.

**11.** The apparatus as claimed in claim 9 or claim 10, further comprising:

> a pulse width modulation (PWM) generator that provides an output signal which provides a drive voltage for the pump element (44) and which receives a control signal that selectively disconnects the pump element (44) from the drive voltage.

**12.** The apparatus as claimed in any one of claims 10 to 12, further comprising:

> the processing unit calculates an electro motive force (EMF) generated by a free-wheeling rotor element and calculates pumping speed responsive to the EMF.

**Patentansprüche**

**1.** Ein Verfahren zum Bestimmen der Flussrate in einem topischen Unterdrucksystem (TNP-System), das die folgenden Schritte beinhaltet:

> Bestimmen einer mit einem Pumpenelement (44) eines TNP-Systems assoziierten Pumpgeschwindigkeit;
> Bestimmen eines Drucks, der mit einem Flussweg assoziiert ist, welcher mit dem Pumpenelement assoziiert ist; und durch Folgendes gekennzeichnet:

> > Bestimmen der Flussrate in dem Flussweg durch Benutzen einer Verweistabelle unter Verwendung einer Beziehung zwischen Flussrate, Pumpgeschwindigkeit und Druck.

**2.** Verfahren gemäß Anspruch 1, das ferner die folgenden Schritte beinhaltet:

> Bestimmen des Drucks durch Messen des Drucks mittels eines Drucksensors.

3. Verfahren gemäß Anspruch 1 oder Anspruch 2, wobei die Flussrate eine Last an dem Pumpenelement (44) beinhaltet.

4. Verfahren gemäß einem der Ansprüche 1 bis 3, wobei der Schritt des Bestimmens der Pumpgeschwindigkeit die folgenden Schritte beinhaltet:

   Bestimmen einer durch ein freilaufendes Element des Pumpenelements (44) erzeugten elektromotorischen Kraft (EMK); und
   Berechnen der Pumpgeschwindigkeit als Reaktion auf die erzeugte EMK.

5. Verfahren gemäß einem der vorhergehenden Ansprüche, das ferner die folgenden Schritte beinhaltet:

   Bestimmen, ob die bestimmte Flussrate innerhalb eines vorbestimmten Betriebsbereichs liegt; und
   Erzeugen eines hörbaren und/oder sichtbaren Alarmhinweises, falls die bestimmte Flussrate eine obere Bereichsgrenze überschreitet oder unter eine untere Bereichsgrenze fällt.

6. Verfahren gemäß einem der Ansprüche 1 bis 5, das ferner die folgenden Schritte beinhaltet:

   zeitweises Variieren der Pumpengeschwindigkeit als Reaktion auf die bestimmte Flussrate;
   Neubestimmen des Drucks im Anschluss an jeden Schritt des Variierens der Pumpengeschwindigkeit; und
   Neubestimmen der Flussrate in dem Flussweg.

7. Verfahren gemäß einem der vorhergehenden Ansprüche, wobei der Schritt des Bestimmens der Flussrate die folgenden Schritte beinhaltet:

   Bestimmen der Flussrate an einem Einlassort des Pumpenelements (44).

8. Verfahren gemäß einem der vorhergehenden Ansprüche, das ferner das Bestimmen der Flussrate in dem Flussweg durch die folgenden Schritte beinhaltet:

   Bestimmen einer Pumpengeschwindigkeit oder eines Drucks;
   Indexieren der Verweistabelle als Reaktion auf die bestimmte Pumpengeschwindigkeit oder den bestimmten Druck; und
   Bestimmen einer Flussrate durch Indexieren eines Flussratenwertes, der mit einem Wert eines Restes der Pumpengeschwindigkeit oder des Drucks in der indexierten Verweistabelle assoziiert ist.

9. Eine Vorrichtung zur Bestimmung der Flussrate in einem topischen Unterdrucksystem (TNP-System), die Folgendes beinhaltet:

   ein Pumpenelement (44), das ein Rotorelement beinhaltet, welches einen Unterdruck in einem Flussweg bereitstellt;
   einen Drucksensor, der eingerichtet ist, um einen Druck in dem Flussweg zu bestimmen; und
   **dadurch gekennzeichnet, dass** sie ferner Folgendes beinhaltet:

   eine Verarbeitungseinheit, die eine mit dem Pumpenelement (44) assoziierte Pumpgeschwindigkeit bestimmt und durch Benutzen einer Verweistabelle unter Verwendung einer Beziehung zwischen Flussrate, Pumpgeschwindigkeit und Druck die Flussrate in dem Flussweg bestimmt.

10. Vorrichtung gemäß Anspruch 9, wobei der Flussweg Folgendes beinhaltet:

    ein Verbindungsrohr, das das Pumpenelement (44) mit einem Behälter (22) des TNP-Systems verbindet; und
    ein Ansaugrohr, das mit dem Behälter (22) verbunden ist und an einer Wundstelle positioniert werden kann.

11. Vorrichtung gemäß Anspruch 9 oder Anspruch 10, die ferner Folgendes beinhaltet:

    einen Pulsdauermodulationsgenerator (PDM-Generator), der ein Ausgangssignal bereitstellt, welches eine Treiberspannung für das Pumpenelement (44) bereitstellt, und
    der ein Steuersignal empfängt, welches das Pumpenelement (44) selektiv von der Treiberspannung trennt.

12. Vorrichtung gemäß einem der Ansprüche 10 bis 12, die ferner Folgendes beinhaltet:

    Die Verarbeitungseinheit berechnet eine durch ein freilaufendes Rotorelement erzeugte elektromotorische Kraft (EMK) und berechnet die Pumpgeschwindigkeit als Reaktion auf die EMK.

**Revendications**

1. Une méthode de détermination du débit dans un système à pression négative topique (TNP), comprenant les étapes consistant à :

déterminer une vitesse de pompage associée à un élément formant pompe (44) d'un système de TNP ;
déterminer une pression associée à une voie d'écoulement associée à l'élément formant pompe ; et **caractérisée par** le fait de
déterminer le débit dans la voie d'écoulement en se servant d'une table à consulter utilisant une relation entre le débit, la vitesse de pompage et la pression.

2. La méthode telle que revendiquée dans la revendication 1, comprenant en sus les étapes consistant à :

déterminer la pression en mesurant la pression à l'aide d'un capteur de pression.

3. La méthode telle que revendiquée dans la revendication 1 ou la revendication 2 dans laquelle le débit comprend une charge sur l'élément formant pompe (44).

4. La méthode telle que revendiquée dans l'une quelconque des revendications 1 à 3 dans laquelle l'étape consistant à déterminer la vitesse de pompage comprend les étapes consistant à :

déterminer une force électromotrice (FEM) générée par un élément en roue libre de l'élément formant pompe (44) ; et
calculer la vitesse de pompage sensible à la FEM générée.

5. La méthode telle que revendiquée dans n'importe quelle revendication précédente, comprenant en sus les étapes consistant à :

déterminer si le débit déterminé est dans les limites d'une plage de fonctionnement prédéterminée ; et
générer un signal d'alarme audible et/ou visible si le débit déterminé dépasse une limite de plage supérieure ou tombe en dessous d'une limite de plage inférieure.

6. La méthode telle que revendiquée dans l'une quelconque des revendications 1 à 5, comprenant en sus les étapes consistant à :

de temps à autre, faire varier la vitesse de pompe sensible au débit déterminé ;
déterminer à nouveau la pression suite à chaque étape consistant à faire varier la vitesse de pompe ; et
déterminer à nouveau le débit dans la voie d'écoulement.

7. La méthode telle que revendiquée dans n'importe quelle revendication précédente dans laquelle l'étape consistant à déterminer le débit comprend les étapes consistant à :

déterminer le débit à l'emplacement d'un orifice d'admission de l'élément formant pompe (44).

8. La méthode telle que revendiquée dans n'importe quelle revendication précédente, comprenant en sus le fait de déterminer le débit dans la voie d'écoulement par les étapes consistant à :

déterminer une vitesse de pompe ou une pression ;
indexer la table à consulter sensible à la vitesse de pompe ou à la pression déterminées ; et
déterminer un débit en indexant une valeur de débit associée à une valeur d'un reste de la vitesse de pompe ou de la pression dans la table à consulter indexée.

9. Appareil pour la détermination du débit dans un système à pression négative topique (TNP), comprenant :

un élément formant pompe (44), comprenant un élément formant rotor qui fournit une pression négative dans une voie d'écoulement ;
un capteur de pression arrangé pour déterminer une pression dans la voie d'écoulement ; et **caractérisé en ce qu'**il comprend en sus
une unité de traitement qui détermine une vitesse de pompage associée à l'élément formant pompe (44) et détermine le débit dans la voie d'écoulement en se servant d'une table à consulter utilisant une relation entre le débit, la vitesse de pompage et la pression.

10. L'appareil tel que revendiqué dans la revendication 9 dans lequel la voie d'écoulement comprend :

un tube de raccordement raccordant l'élément formant pompe (44) à un contenant (22) du système de TNP ; et
un tube d'aspiration raccordé au contenant (22) et pouvant être placé au niveau du site d'une plaie.

11. L'appareil tel que revendiqué dans la revendication 9 ou la revendication 10, comprenant en sus :

un générateur à modulation de largeur d'impulsion (MLI) qui fournit un signal de sortie qui fournit une tension d'entraînement pour l'élément formant pompe (44) et qui reçoit un signal de commande qui déconnecte sélectivement l'élément formant pompe (44) de la tension d'entraînement.

**12.** L'appareil tel que revendiqué dans l'une quelconque des revendications 10 à 12, comprenant en sus :

l'unité de traitement calcule une force électromotrice (FEM) générée par un élément formant rotor en roue libre et calcule la vitesse de pompage sensible à la FEM.

FIG. 1

EP 2 175 907 B1

FIG. 2

FIG. 3

EP 2 175 907 B1

FIG. 4

288,260

272

204

208

202

206

222

FIG. 5

218

302

302

214

264

216

302

212

302

226

FIG. 6

FIG. 7

FIG. 8

FIG. 9

1004

1003

$V_{set}$

1002

1001

1000

FIG. 10

FIG. 11

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004037334 A
- WO 2005046760 A
- WO 2005105180 A
- GB 2307180 A
- US 20070016152 A